# FASCICULE DE BREVET EUROPEEN

(11) **EP 2 114 248 B1**
(45) Date de publication et mention de la délivrance du brevet: **23.11.2016**
(21) Numéro de dépôt: 07820940.0
(22) Date de dépôt: 04.10.2007
(51) Int. Cl.: A61B 5/103, A61B 5/0488

(54) **DISPOSITIF DE DETECTION ET DE SUIVI D'UNE LESION DU LIGAMENT CROISE ANTERIEUR DU GENOU**
VERFAHREN ZUM NACHWEIS UND ZUR ÜBERWACHUNG VON VERLETZUNGEN DES VORDEREN KREUZBANDS IM KNIE
METHOD FOR DETECTING AND MONITORING INJURIES OF THE KNEE ANTERIOR CRUCIATE LIGAMENT

(30) Priorité: 05.10.2006 FR 0608725; 05.10.2006 FR 0608726
(43) Date de publication de la demande: 11.11.2009
(73) Titulaire: Genourob, 53500 Montenay (FR)
(72) Inventeur: NOUVEAU, Stéphane, 53500 Ernee (FR); ROBERT, Henri, 53100 Mayenne (FR)
(74) Mandataire: Benech, Frédéric
(86) Numéro de dépôt international: PCT/EP2007/060563
(87) Numéro de publication internationale: WO 2008/040790

(56) Documents cités:
- EP-A- 0 568 148
- WO-A-02/38047
- DE-A1- 3 925 014
- US-A- 4 969 471
- US-A1- 4 534 364
- FELLER J ET AL: "EMG biofeedback assisted KT-1000 evaluation of anterior tibial displacement." KNEE SURGERY, SPORTS TRAUMATOLOGY, ARTHROSCOPY : OFFICIAL JOURNAL OF THE ESSKA 2000, vol. 8, no. 3, 2000, pages 132-136, XP002426444 ISSN: 0942-2056

## Description

Le domaine de l'invention est celui des techniques de détection d'une lésion d'un ligament d'un membre. Plus précisément, l'invention concerne un appareil permettant de mesurer la translation antérieure du tibia par rapport au fémur, de façon à mettre en évidence une lésion du ligament croisé antérieur (LCA) du genou.

Dans le domaine de l'invention, plusieurs techniques ont été proposées par l'art antérieur.

On connaît notamment une première technique mettant en oeuvre un appareil connu sous l'appellation « TELOS » (marque déposée). Selon cette technique, le patient est placé en décubitus latéral sur une table radiographique, et un cliché est effectué au niveau du genou (ce dernier étant soumis à une contrainte exercée sur la face postérieure du mollet, en vue de reproduire ainsi le tiroir antérieur).

Le « TELOS » est un appareil qui permet une radiographie dynamique mécanique. Il est constitué d'un bâti central métallique, sur lequel le membre est positionné avec le genou fléchi à 25°, avec la cuisse bloquée à la face antérieure (appui sus-rotulien) ainsi qu'à la face antéro-inférieure du tibia.

Une poussée postérieure doit être exercée à hauteur du rebord tibial postérieur en radioscopie télévisée. Deux forces de 150 N puis de 250 N sont appliquées et visualisées au dynamomètre.

Deux clichés sont ensuite réalisés : un du membre lésé et un de l'autre membre. Une comparaison des deux clichés est effectuée (c'est ce différentiel qui est analysé). Sur les clichés, on trace une première ligne parallèle aux plateaux tibiaux, et une deuxième ligne perpendiculaire à la première et passant par le bord postérieur soit des condyles, soit des plateaux tibiaux. La distance entre les deux lignes est mesurée (en mm), ce qui donne le tiroir antérieur du tibia par rapport au fémur.

Cette technique s'avère en pratique objective, mais présente toutefois plusieurs inconvénients.

Tout d'abord, un premier inconvénient réside dans le recours au rayonnement X qui empêche des mesures itératives dans le cadre d'un suivi du patient, que ce soit en pré-opératoire ou post-lésionnel, ou encore en postopératoire pour suivre l'évolution de la ligamentoplastie par exemple.

Un autre inconvénient réside dans le fait de devoir placer les deux membres de façon très rigoureuse. En effet, le placement du membre lésé et celui du membre non lésé doivent être identiques des deux côtés : en particulier, les condyles doivent être superposés (sous radioscopie), ainsi que les plateaux tibiaux, et le membre doit être en position neutre de rotation.

Par ailleurs, le « TELOS » est réputé pour sa faible sensibilité, et donc son manque de précision, en raison des contractures réflexes des muscles ischio-jambiers lors de la poussée (du fait de la position peu confortable et souvent douloureuse pour le patient), qui ne permettent pas d'obtenir de bonnes mesures de la translation antérieure du tibia (taux de faux-négatifs élevé).

On connaît également une autre technique mettant en oeuvre un appareil connu sous l'appellation « KT 1000 » (marque déposée).

Cet appareil comprend :
- un ensemble support de la jambe, se décomposant en une partie supportant la cuisse et une partie réglable en longueur et destinée à s'étendre entre le genou et la cheville, l'angle entre les deux parties étant réglable ;
- un moyen de poussée sur le mollet comprenant une partie sur laquelle le mollet est destiné à venir en appui, et relié à une poignée destinée à être manipulée par un opérateur, cette poignée intégrant un mécanisme de réglage de la force appliquée au moyen de poussée ;
- un dispositif de mesure de la translation antérieure du tibia par rapport au fémur.

La mise en oeuvre de cet appareil consiste à fixer celui-ci sur le devant de la jambe (le patient étant en décubitus dorsal), par sanglage à l'aide de bandes auto-agrippantes, puis à tirer sur la poignée de l'appareil afin d'exercer une traction sur le tibia, la cuisse (le fémur) étant maintenue par un appui rotulien, le genou étant positionné à 20° de flexion et le pied étant en position neutre à l'aide d'une cale.

Les avantages de cet appareil est qu'il est facilement transportable et qu'il est d'une mise en oeuvre très rapide.

En revanche, il présente plusieurs inconvénients.

Un inconvénient majeur de cet appareil réside dans le fait que les résultats obtenus sont étroitement liés aux manipulations de l'appareil par l'opérateur. En d'autres termes, un même opérateur obtiendra des résultats identiques à différentes mesures sur un même patient présentant la même lésion seulement en effectuant des manipulations de l'appareil strictement de la même manière.

Bien entendu, une telle constance et une telle régularité dans les manipulations sont réservées à des opérateurs expérimentés.

Cela étant, cet inconvénient se reporte de façon sensible lorsqu'il s'agit de comparer les mesures effectuées par plusieurs opérateurs. Dans la pratique, on constate à ce sujet que peu de corrélation des données recensées sont réalisées.

Par ailleurs, un tel appareil ne s'affranchit pas des contractions musculaires parasites susceptibles d'intervenir au cours des mesures, ce qui impacte bien entendu la fiabilité de l'appareil.

US 4534364A1, WO02/38047A, US4969471A, DE3925014A1 et EP0568148A montrent d'autres dispositifs de diagnose du tibia.

De façon générale, les techniques de l'art antérieur présentent les inconvénients suivants :
- les résultats obtenus sont dépendants de la qualité et/ou de la précision des manipulations réalisées par les opérateurs ;
- la précision des résultats n'est que de l'ordre du millimètre ;
- les instruments ne permettent pas une standardisation du positionnement du membre lors des enregistrements ;
- il n'est pas tenu compte de l'état de relaxation musculaire de la cuisse.

L'invention a notamment pour objectif de pallier les inconvénients de l'art antérieur.

Plus précisément, l'invention a pour objectif de proposer une technique de détection et de suivi d'une lésion du ligament croisé antérieur du genou qui soit plus fiable que les techniques de l'art antérieur.

L'invention a également pour objectif de fournir une telle technique qui permette d'obtenir des résultats avec une plus grande précision que celle obtenue avec les techniques de l'art antérieur.

L'invention a aussi pour objectif de fournir une telle technique qui soit peu ou pas dépendante de la qualité et/ou de la régularité des manipulations faites par un opérateur.

Un autre objectif de l'invention est de fournir une telle technique qui permette d'envisager un suivi fiable d'un même patient et/ou de corréler des coordonnées recensées dans des conditions satisfaisantes.

Encore un autre objectif de l'invention est de fournir une telle technique qui soit pratique d'utilisation.

L'invention a encore pour objectif de fournir une telle technique qui soit simple de conception et facile à mettre en oeuvre.

Ces objectifs, ainsi que d'autres qui apparaîtront par la suite, sont atteints grâce à l'invention qui a pour objet un dispositif de détection et de suivi d'une lésion (rupture ou déchirure) du LCA par translation antérieure du tibia par rapport au fémur comportant un support du membre inférieur caractérisé en ce qu'il comprend un moyen de poussée de la face postéro-supérieure du mollet et d'un capteur de déplacement du tibia venant se positionner sur la face antéro-supérieure du tibia, sur la TTA (Tubérosité Tibia Antérieure).

Selon une caractéristique avantageuse, le dispositif comporte au moins une électrode de surface en regard des muscles ischio-jambiers et une de référence au niveau du bord latéral ou médial du genou permettant de détecter toute contraction musculaire parasite qui empêche de déterminer avec exactitude la mesure du déplacement antérieur du tibia.

De cette façon, le dispositif prend en considération les contractions musculaires parasites susceptibles de nuire à la qualité des mesures effectuées. A tout le moins, comme cela va apparaître plus clairement par la suite, il est possible, grâce à cette détection, d'effectuer ou pas la mesure en fonction du relâchement musculaire de la cuisse.

Selon une solution préférée, ledit moyen de poussée comprend au moins un vérin piloté par un calculateur de poussée.

Ainsi, la contrainte exercée sur le mollet est pilotée de façon très précise, ce qui, bien entendu, impacte de façon positive la précision des résultats obtenus. D'autre part, la contrainte exercée est ainsi indépendante de toute intervention humaine, ce qui contribue à augmenter notablement la fiabilité du dispositif.

En particulier, un dispositif ainsi constitué permet de :
- exercer des contraintes reproductibles pour un même patient, dans le cadre d'un suivi du patient ;
- corréler des données obtenues par différents opérateurs.

Préférentiellement, le dispositif comprend un capteur de pression destiné à mesurer la pression exercée par ledit membre sur ledit support, ledit calculateur étant relié audit capteur de pression de façon à piloter ledit vérin en fonction de données fournies par ledit capteur de pression.

On obtient ainsi une solution simple et efficace pour piloter précisément la poussée exercée par le vérin sur la face postéro-supérieure du mollet. De plus, on comprend que de cette façon, les données représentatives de la poussée sont centralisées au niveau du calculateur de pilotage du vérin.

Selon une solution avantageuse, ledit capteur de déplacement fournit des données sous forme électronique audit calculateur.

De cette façon, on peut obtenir une mesure de la translation antérieure du tibia par rapport au fémur avec une précision de l'ordre du dixième de millimètre. On peut donc réaliser une analyse avec une plus grande finesse que les analyses obtenues avec les solutions de l'art antérieur, et mieux répertorier le type de lésion du LCA (déchirure partielle d'un faisceau ou de l'ensemble...).

Selon l'invention le dispositif comprend les moyens de serrage dudit membre sur ledit support, lesdits moyens de serrage comprenant des moyens de mesure et/ou de repérage d'une force de serrage. Selon une solution avantageuse, lesdits moyens de serrage comprennent des moyens de sanglage du fémur dudit membre sur ledit support, au moins un capteur de pression étant préférentiellement placé sur ledit support au voisinage de ladite sangle de façon à procurer une mesure de force d'appui dudit membre sur ledit support.

Le serrage de la cuisse lors des mesures ayant une influence non négligeable sur la qualité et/ou la précision des résultats obtenus, un dispositif de détection de suivi de lésion ainsi constitué permet d'effectuer un serrage précis. De plus, la force de serrage fournie par le capteur de pression est une donnée pouvant aisément être extraite, en l'occurrence sous forme électronique, puis mémorisée, ceci contribuant à assurer des conditions de mesures reproductibles.

Avantageusement, le dispositif comprend une coque de maintien quadricipital reliée par au moins deux sangles audit support.

Une telle coque permet de transmettre efficacement la force de serrage au membre du patient. Bien entendu, une telle coque pourra être adaptée à la morphologie des patients (par exemple en prévoyant une coque pour des patients adultes et une coque pour des patients enfants).

Selon une autre caractéristique de l'invention, lesdits moyens de serrage comprennent des moyens de sanglage du pied dudit membre, lesdits moyens de sanglage du pied étant préférentiellement montés coulissants sur ledit support de façon à pouvoir être écartés/rapprochés desdits moyens de sanglage du fémur.

Le maintien en position du membre sur le support peut ainsi être réalisé de façon ajustée à la morphologie du patient.

Préférentiellement, lesdits moyens de sanglage du pied portent des moyens de repérage du niveau de serrage.

A nouveau, une telle caractéristique contribue à mémoriser des conditions dans lesquelles les mesures sont réalisées.

Selon une solution avantageuse, le dispositif comporte des moyens de mise en flexion (x) du genou dudit membre permettant d'avoir : 15° < x < 25°, ledit dispositif comprenant préférentiellement une partie support de cuisse et une partie support de mollet reliées entre elles, et présentant respectivement une extrémité arrière et une extrémité avant, au moins ladite extrémité avant présentant des moyens de réglage en hauteur.

On peut de cette façon procéder aux mesures dans des conditions de flexion opératoires classiques, quelles que soient les caractéristiques dimensionnelles du membre étudié.

Selon une autre caractéristique avantageuse de l'invention, ledit capteur de déplacement est fixé sur une potence montée sur le support de manière coulissante et articulée.

Le positionnement du capteur peut donc être de cette façon ajusté par rapport au membre étudié.

Selon encore une autre caractéristique avantageuse de l'invention, lesdites électrodes sont reliées à des moyens de traitement couplé audit calculateur, lesdits moyens de traitement étant destinés à provoquer une neutralisation dudit moyen de poussée en cas de détection de contractions musculaires par ladite ou lesdites électrodes.

De cette façon, on évite de procéder à une mesure en cas de présence de contractions musculaires. En d'autres termes, on s'assure de cette façon que les résultats obtenus lors des mesures effectuées ne sont pas faussés par d'éventuelles contractions musculaires parasites.

Avantageusement, lesdites électrodes sont reliées à des moyens d'affichage de ladite contraction musculaire.

Il est ainsi possible pour le patient de visualiser le niveau de son relâchement musculaire, ou à tout le moins de visualiser s'il est suffisant pour permettre la mesure par le dispositif.

D'autres caractéristiques et avantages de l'invention apparaîtront plus clairement à la lecture de la description suivante d'un mode de réalisation préférentiel de l'invention, donné à titre d'exemple illustratif et non limitatif, et des dessins annexés parmi lesquels :
- la figure 1 est une vue de côté d'un dispositif selon l'invention ;
- la figure 2 est une vue de côté d'un dispositif selon l'invention sur lequel un membre à étudier est présent.

Tel qu'indiqué précédemment, le principe de l'invention réside dans le fait de proposer un dispositif de détection et de suivi d'une lésion du LCA du genou comprenant un moyen de poussée de la face postéro-supérieure du mollet et un capteur de déplacement du tibia, comprenant en outre préférentiellement :
- des moyens de positionnement et de maintien du membre sur le support conçu de façon à permettre la mesure et le paramétrage des conditions de positionnement et de maintien du membre sur le support ;
- des moyens de détection et de prise en compte d'une contraction musculaire parasite.

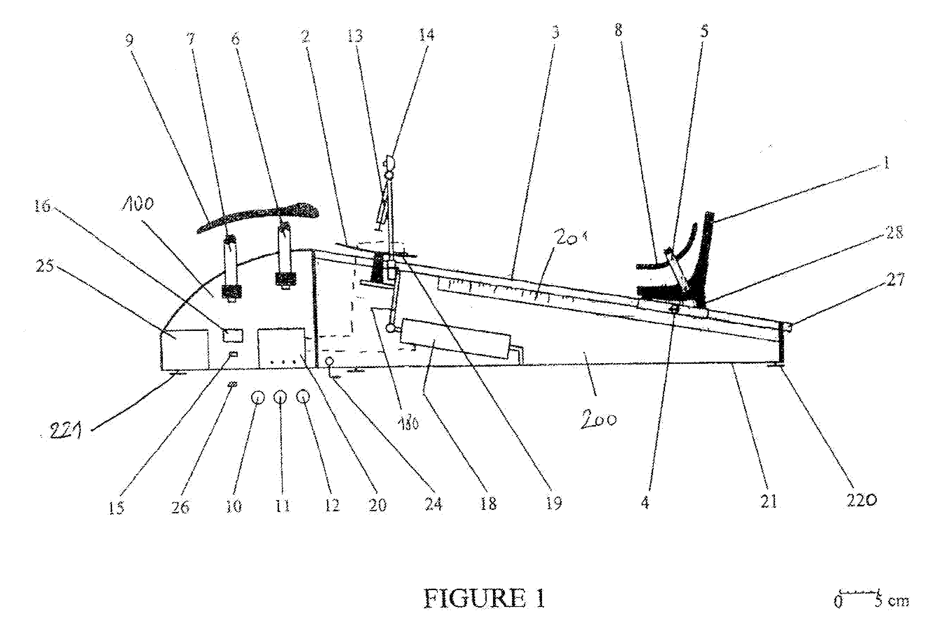

En référence à la figure 1, un dispositif selon l'invention comprend :
- une partie support 100 pour la cuisse du membre à étudier ;
- une partie support 200 pour le mollet du membre à étudier, les parties 100 et 200 du support étant solidaires l'une de l'autre.

On comprend que les parties 100 et 200 forment ensemble un support pour l'ensemble du membre inférieur à étudier.

La partie support de mollet 200 présente en son extrémité avant (constituant également l'extrémité avant du dispositif) un ou plusieurs pieds 220 réglables en hauteur. Parallèlement, la partie support de cuisse 100 présente en son extrémité arrière (constituant également l'extrémité arrière du dispositif) un ou plusieurs pieds 221 réglables en hauteur.

A titre indicatif, les parties 100 et 200 sont montées sur un socle 21 réalisé en un matériau inoxydable, formé par exemple d'une tôle pliée, présentant des évidements en vue d'alléger le dispositif Le socle présente une longueur de 60 cm, une largeur de 20 cm et une hauteur de 15 cm à l'une de ses extrémités et de 5 cm à l'autre de ses extrémités. Des patins 22 réglables en hauteur sont rapportés sous le support.

Une partie en matériau composite 3 (long et large de 20 cm) s'adapte au socle 21 par un système d'attache rapide. A l'intérieur de cette partie, toute la partie électrique et électronique est insérée (microcalculateur, câblage, cadran d'affichage, ...) et à l'extérieur, un moulage permet de recevoir la face postérieure de la cuisse.

Selon plusieurs caractéristiques avantageuses du dispositif selon l'invention, celui-ci comprend :
- des moyens de poussée de la face postéro-supérieure du mollet ;
- des moyens de mesure du déplacement du tibia, lorsqu'une contrainte est exercée par des moyens de poussée ;
- des moyens de serrage du membre sur le support.

Selon le présent mode de réalisation, les moyens de serrage du membre sur le support comprennent :
- des moyens de serrage du fémur ;
- des moyens de serrage du pied.

Les moyens de serrage du fémur comprennent une coque quadricipitale 9 reliée par un ou plusieurs sangles 6, 7 à la partie support 100 du dispositif.

Parallèlement, la partie support 200 porte un socle 2 sur lequel est monté un capteur de pression 19 (du type de celui commercialisé sous le nom de "Flexiforce type 1230 (marque déposée)), ce capteur étant relié à un microprocesseur 20 (de type PIC).

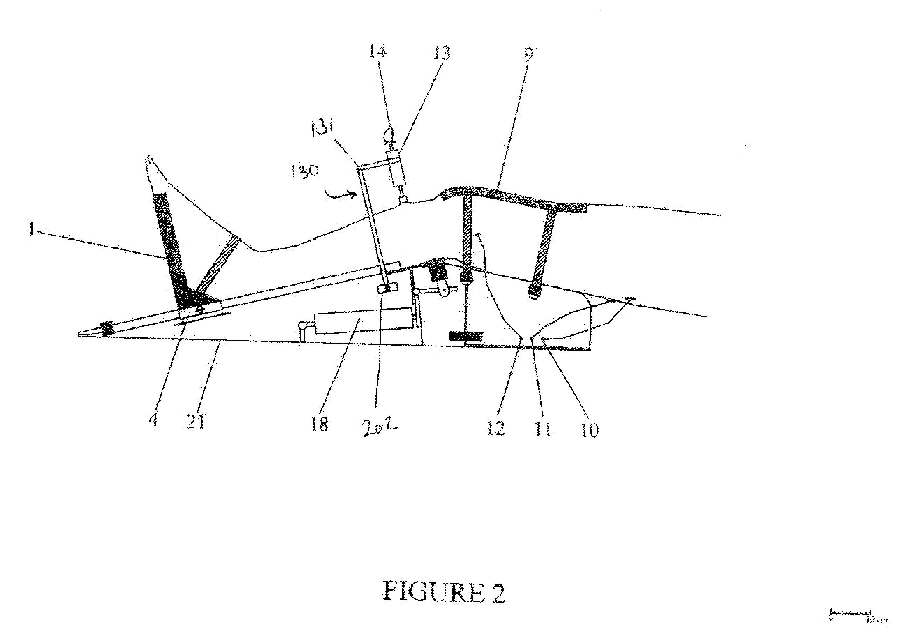

Ainsi, lorsque le membre du patient est rapporté sur le dispositif dans une position telle que celle illustrée par la figure 2, la coque 9 est placée de façon à recouvrir la rotule et le bas de la cuisse, et les sangles 6, 7 sont serrées. Le capteur de pression 19 fournit alors une donnée au microprocesseur 20 indiquant la force de serrage, cette donnée étant destinée à être mémorisée dans un registre dédié au patient de telle sorte que cette donnée puisse être utilisée lors d'une mesure ultérieure pour effectuer un serrage de la cuisse avec une force de serrage identique.

En effet, pour pouvoir opérer un suivi fiable d'un patient, il est nécessaire que la pression de serrage initiale soit identique d'une mesure à l'autre pour le même patient (critère de reproductibilité des conditions de mesure).

Les moyens de serrage du membre comprennent également des moyens de sanglage du pied.

Selon le mode de réalisation illustré par les figures 1 et 2, les moyens de serrage comprennent :
- une coque thermoformée 1 destinée à recevoir un pied
- une coque 8 destinée à être placée sur le coup de pied ;
- une sangle de serrage 5 reliant les coques 1 et 8.

La coque 1 est montée coulissante sur des tubes 3 portés par la partie support 200, une clé de serrage rapide 4 permettant l'immobilisation de la coque 1 sur les tubes 3.

On note que la coque 1 est couplée à une articulation 28 de telle sorte que la coque puisse être pivotée de façon à imposer une rotation interne ou externe au pied.

Selon un autre mode de réalisation envisageable, il est possible de proposer un dispositif sans la coque 1. Les moyens de sanglage du pied sont alors constitués par une sangle, et éventuellement une coque de coup de pied, montée directement sur des supports eux-mêmes montés coulissants sur la partie support 200.

On comprend que, ainsi constitué (avec ou sans la coque 1), le dispositif permet un réglage de la position de la sangle 5 sur la longueur de la partie support 200, de façon à adapter le dispositif à la longueur du membre, ceci en faisant varier la position de la coque 1 par rapport au socle 2 et/ou par rapport aux moyens de serrage du fémur contre la partie 100 (par l'intermédiaire de la coque 9 et des sangles 6, 7). On peut donc, en fonction de la grandeur du membre, adapter la position du pied sur l'appareil de façon à ce que le bord postéro-supérieur du tibia soit au contact du bord supérieur du socle 2.

De plus, les moyens de sanglage du pied portent des moyens de repérage du niveau de serrage, et sont constitués selon le présent mode de réalisation par des sangles à attache rapide du type de celles utilisées dans le domaine des chaussures de ski. Les sangles de serrage du pied sont marquées de 5 mm en 5mm.

En outre, une réglette graduée 201 est rapportée sur la partie support 200, de telle sorte que la position du pied puisse être repérée et mémorisée.

Selon une autre caractéristique de l'invention, le moyen de poussée de la face postéro-supérieure du mollet comprend :
- un socle 2, mentionné précédemment ;
- un vérin 18, piloté par le calculateur 20.

Une fois le membre placé sur le dispositif, le bord postéro-supérieur du tibia étant en contact avec le socle 2, le capteur de pression 19 sert dans un premier temps à communiquer au microprocesseur des données sous forme électronique représentatives du serrage. Lorsque le socle 2 exerce une poussée sur le mollet sous l'effet du vérin 18 par l'intermédiaire d'une tringlerie 180, le capteur 19 fournit alors au microprocesseur 20 de nouvelles informations représentatives de la force de poussée sur le mollet. Il est donc aisé de programmer le calculateur de façon à ce que celui-ci pilote le vérin de façon à exercer des poussées préprogrammées, par exemple de 67 N, 89 N et 134 N.

Par ailleurs, la partie support 200 porte une potence 130 susceptible d'être coulissée à l'intérieur d'une glissière 202 ménagée dans la partie support 200 et s'étendant de façon à pouvoir faire varier la position de la potence par rapport à la longueur de la partie support 200. Cette potence présente en outre, une articulation 131.

Un capteur de déplacement 13 est monté à l'extrémité libre de la potence 130, la position du capteur 13 pouvant être réglée par coulissement de la potence dans la glissière 202 et/ou grâce à l'articulation 131.

Le capteur de déplacement 13 est du type de celui commercialisé sous le nom "Novotechnik" (marque déposée).

La position du capteur 13 peut également être modifiée par rapport à la potence 130, dans une direction normale à la surface du membre sur laquelle le capteur est destiné à être mis en appui.

Le réglage est effectué de telle sorte que le genou soit en flexion avec un angle compris entre 15° et 25°, 1

Selon une caractéristique particulièrement avantageuse de l'invention, le dispositif comporte également :
- deux électrodes 10, 11 de surface destinées à être placées sur le membre en regard des muscles ischio-jambiers ;
- une électrode de référence 12 destinée à être placée sur le membre sur le bord médial ou sur le bord latéral du genou.

Ces électrodes sont destinées à détecter toute contraction musculaire parasite susceptible d'empêcher de déterminer avec exactitude la mesure du déplacement antérieur du tibia (amplitude totale). En effet, le déplacement réel du tibia ne peut s'effectuer qu'au moment du relâchement des muscles ischio-jambiers (ceci correspondant à un certain seuil d'activité électrique prédéterminé).

Les électrodes 10, 11 et 12 sont reliées au microprocesseur 20 qui traite les données électroniques fournies par ces électrodes, le microcalculateur étant programmé pour neutraliser le déclenchement des moyens de poussée si une contraction musculaire est détectée par les électrodes 10, 11 et 12.

De plus, des diodes 14 sont reliées au calculateur 20, de façon à fournir une indication visuelle du niveau de relâchement musculaire au niveau des ischio-jambiers. Tel qu'illustré par les figures 1 et 2, ces diodes 14 sont positionnées au-dessus du capteur 13, de telle sorte qu'elles soient orientées de façon aisément visibles pour le patient, les diodes procurant alors un effet de biofeedback : plus il y a de diodes à s'éteindre, plus le relâchement est important.

Les électrodes 10, 11 sont destinées à être positionnées sur la partie moyenne (tiers moyen) de la face postérieure de la cuisse en regard des muscles ischio-jambiers (groupe latéral et groupe médial). L'électrode de référence 12 est destinée quant à elle à être située soit sur le bord médial soit sur le bord latéral du genou (proche d'un bord osseux).

On note que l'élément de contact des électrodes 10, 11 et 12 est destiné à être changé après chaque patient (les mêmes électrodes pouvant toutefois être utilisées pour les mesures sur les deux membres d'un même patient).

Le mode opératoire d'un dispositif selon l'invention tel que celui décrit précédemment est explicité ci-après.

Le patient est allongé en décubitus dorsal (position la plus confortable et idéale pour le relâchement musculaire), et pose sa jambe sur l'appareil en posant son pied en position neutre de rotation.

Dans un premier temps, le membre du patient présentant la lésion est placé sur le dispositif. La position des moyens de serrage du pied sur la partie support 200 est ajustée à la longueur du membre. Le pied est ensuite sanglé à l'aide de la sangle 5. La position de la sangle 5 du pied le long de la réglette 201 est repérée et mémorisée.

Les surfaces d'appui de la partie support de cuisse 100 et de la partie support de mollet 200 présentent des inclinaisons l'une par rapport à l'autre de telle sorte que, en moyenne, un membre positionné sur le dispositif présente une flexion du genou comprise entre 12° et 25°. Toutefois, pour des petits membres (tels ceux d'un enfant) ou des membres de grandes tailles (tels ceux d'une personne dont la taille avoisine 2 mètres de haut), le genou peut présenter une flexion qui n'entre pas dans cette plage. Dans ce cas, on peut amener le genou dans la plage de 15° à 25° en intervenant (par vissage/dévissage) sur le réglage en hauteur des pieds 220 et 221. Ainsi, pour un angle inférieur à 15°, on augmente la hauteur de l'extrémité arrière de la partie support 100 par rapport à celle de l'extrémité avant de la partie support 200. Inversement, pour un angle supérieur à 25°, on augmente la hauteur de l'extrémité avant de la partie support 200 par rapport à celle de l'extrémité arrière de la partie support 100. L'angle de flexion du genou peut être mesuré avec un goniomètre.

La coque de maintien quadricipital 9 est correctement placée sur le genou et le bas de la cuisse du membre, un serrage du fémur contre la partie support 100 étant exercé en resserrant les sangles 6, 7 reliant la coque 9 à la partie support 100.

A ce stade, le capteur 19 transmet au microcalculateur 20 une information support sous forme électronique représentative du serrage initial du fémur.

Le capteur de déplacement 13 est placé sur le membre, de façon à être positionné en regard de la tubérosité tibiale antérieure. Pour cela, la potence portant le capteur est éventuellement déplacée, et/ou le capteur est orienté par rapport à la potence.

Les électrodes 10, 11 sont alors placées sur la face postérieure de la cuisse, en regard des muscles ischio-jambiers, et l'électrode de référence 12 est placé sur le bord médial (ou latéral) du genou.

Une fois ces opérations réalisées, un bouton de commande 15 (ici représenté sur le dispositif, mais pouvant, en pratique, être constitué par une touche "entrée" sur un ordinateur auquel est relié le dispositif) est actionné, l'analyse de la pression de serrage s'inscrit sur le cadran 16 (ou sur un écran d'ordinateur auquel est relié le dispositif) et peut être modifiée en fonction de la force de serrage souhaitée.

Puis, le microcalculateur 20 pilote le vérin 18 de telle sorte que celui-ci exerce sur le socle 2 une poussée. Le déclenchement du vérin n'est toutefois commandé par le microcalculateur que si le relâchement musculaire mesuré par les électrodes 10, 11 est suffisant. Si une contraction musculaire est détectée, une ou plusieurs des diodes 14 s'allume indiquant au patient qu'il doit tenter d'exercer un relâchement musculaire du membre à étudier.

Si le patient parvient à un relâchement musculaire suffisant, aucune des diodes n'est allumée, et le vérin est actionné par le microcalculateur à différentes pressions préprogrammées (67 N, 89 N et 134 N), qui peuvent être présélectionnées séparément.

Les données relatives aux pressions exercées par le vérin, ainsi que les données procurées par le capteur de déplacement 13 (indiqué en dixième de millimètre) s'inscrivent alors sur le cadran 16.

Le microcalculateur commande alors le retour à l'état initial du vérin.

On note qu'un bouton 26 peut être actionné par l'opérateur dans le cas d'une défaillance du dispositif, stoppant toute activité de ce dernier.

On comprend que le microprocesseur 20 centralise de nombreuses fonctions. En effet, il analyse et/ou pilote les différents paramètres suivants :
- le déclenchement du vérin ;
- la pression exercée par le vérin sur le socle 2 ;
- les données transmises par le capteur de pression 19 ;
- la pression de serrage initiale procurée par le capteur de pression 19 ;
- les contractions musculaires mesurées par les électrodes 10, 11 est 12 ;
- l'éclairage des diodes 14 ;
- les données affichées par le cadran 16 ou sur l'écran d'un ordinateur auquel est relié le dispositif;

On note également que le dispositif peut intégrer une mémoire interne de stockage des données relatives aux mesures effectuées et/ou être connectées à un ordinateur en vue du stockage et du traitement ultérieur des données relatives aux mesures.

La même procédure est effectuée sur la jambe controlatérale afin d'obtenir des mesures de translation, et de les comparer par rapport au membre lésé. C'est ce différentiel qui est analysé afin de montrer s'il existe une lésion ou non du LCA.

On note que le dispositif permet de suivre dans le temps l'évolution des mesures effectuées sur un membre lésé, afin de savoir s'il existe une aggravation ou non de la lésion initiale grâce à des mesures itératives, de mois en mois par exemple.

Le dispositif selon l'invention permet de détecter une lésion du ligament croisé (LCA), que celui-ci soit totalement rompu ou qu'il présente une déchirure partielle. Le dispositif permet d'opérer un suivi régulier du patient et des mesures itératives qui permettent de voir l'évolution de la laxité du genou.

Si la lésion n'est pas totale (déchirure du LCA), elle peut être mise en évidence au fil du temps par les mesures itératives de translation du tibia. En effet, le ligament dégénère (nécrose), très souvent inéluctablement, et ceci entraîne une aggravation de la laxité antérieure du tibia qui se verra lors des prises de mesure à répétition dans le temps.

Un dispositif selon l'invention est particulièrement destiné aux équipes chirurgicales spécialisées dans la ligamentoplastie du LCA, notamment en pré-opératoire pour objectiver avec précision la laxité du genou, ou en postopératoire pour voir l'évolution de cette dernière au fil des mois voire des années, et comparer des techniques opératoires entre elles (laxité plus importante dans le temps pour une technique ou une autre...).

De même, les médecins du sport peuvent utiliser un dispositif selon l'invention en tant qu'outil de dépistage précoce, ou à distance post-lésionnelle ou de suivi des athlètes après intervention chirurgicale.

Un dispositif selon l'invention s'adresse également aux médecins rééducateurs (de médecine physique) et aux masseurs-kinésithérapeutes, qui peuvent alors suivre l'évolution des ligamentoplasties en rééducation et adapter leur traitement en fonction des laxités trouvées.

## Revendications

1. Dispositif de détection et de suivi d'une lésion (rupture ou déchirure) du LCA par translation antérieure du tibia par rapport au fémur comportant un support du membre inférieur, un moyen de poussée (2) de la face postéro-supérieure du mollet et un capteur de déplacement (13) du tibia venant se positionner sur la face antéro-supérieure du tibia, sur la TTA (Tubérosité Tibia Antérieure), **caractérisé en ce qu'**il comprend des moyens de serrage dudit membre sur ledit support, lesdits moyens de serrage comprenant des moyens de mesure et/ou de repérage d'une force de serrage.

2. Dispositif selon la revendication 1, **caractérisé en ce qu'**il comporte au moins une électrode de surface en regard des muscles ischio-jambiers et une de référence au niveau du bord latéral ou médial du genou permettant de détecter toute contraction musculaire parasite qui empêche de déterminer avec exactitude la mesure du déplacement antérieur du tibia.

3. Dispositif selon l'une des revendications 1 et 2, caractérisé en ce ledit moyen de poussée comprend au moins un vérin piloté par un calculateur de poussée.

4. Dispositif selon la revendication 3, **caractérisé en ce qu'**il comprend un capteur de pression destiné à mesurer la pression exercée par ledit membre sur ledit support, ledit calculateur étant relié audit capteur de pression de façon à piloter ledit vérin en fonction de données fournies par ledit capteur de pression.

5. Dispositif selon l'une des revendications 3 et 4, **caractérisé en ce que** ledit capteur de déplacement fournit des données sous forme électronique audit calculateur.

6. Dispositif selon l'une quelconque des revendications précédentes, **caractérisé en ce que** lesdits moyens de serrage comprennent des moyens de sanglage du fémur dudit membre sur ledit support, au moins un capteur de pression étant placé sur ledit support au voisinage de ladite sangle de façon à procurer une mesure de force d'appui dudit membre sur ledit support.

7. Dispositif selon la revendication 6, **caractérisé en ce qu'**il comprend une coque de maintien quadricipital (9) reliée par au moins deux sangles audit support.

8. Dispositif selon l'une quelconque des revendications précédentes, **caractérisé en ce que** lesdits moyens de serrage comprennent des moyens de sanglage du pied dudit membre.

9. Dispositif selon la revendication 8, **caractérisé en ce que** lesdits moyens de sanglage du pied sont montés coulissants sur ledit support de façon à pouvoir être écartés/rapprochés desdits moyens de sanglage du fémur.

10. Dispositif selon la revendication 9, **caractérisé en ce que** ledit support présente des graduations le long desquels lesdits moyens de sanglage du pied sont montés coulissants.

11. Dispositif selon l'une des revendications 8 à 10, **caractérisé en ce que** lesdits moyens de sanglage du pied portent des moyens de repérage du niveau de serrage.

12. Dispositif selon l'une quelconque des revendications précédentes, **caractérisé en ce qu'**il comporte des moyens de mise en flexion (x) du genou dudit membre permettant d'avoir : 15[deg.] < x < 25[deg.], ledit dispositif comprenant préférentiellement une partie support de cuisse et une partie support de mollet reliées entre elles, et présentant respectivement une extrémité arrière et une extrémité avant, au moins ladite extrémité avant présentant des moyens de réglage en hauteur.

13. Dispositif selon l'une quelconque des revendications prédédentes, **caractérisé en ce que** ledit capteur de déplacement (13) est fixé sur une potence montée sur le support de manière coulissante et articulée.

14. Dispositif selon l'une quelconque des revendications 3 à 13 dépendantes de la revendication 2, **caractérisé en ce que** lesdites électrodes sont reliées à des moyens de traitement couplés audit calculateur, lesdits moyens de traitement étant destinés à provoquer une neutralisation dudit moyen de poussée en cas de détection de contraction musculaire par ladite ou lesdites électrodes.

15. Dispositif selon l'une quelconque des revendications 3 à 14 dépendantes de la 2, **caractérisé en ce que** lesdites électrodes sont reliées à des moyens d'affichage de ladite contraction musculaire.

## Patentansprüche

1. Vorrichtung zum Erfassen und zur Überwachung einer Verletzung des vorderen Kreuzbands (Bruch oder Riss) durch vordere Translation des Schienbeins in Bezug auf den Oberschenkelknochen, umfassend eine Stütze für das untere Glied, ein Mittel (2) zum Schieben der oberen hinteren Seite der Wade und einen Sensor (13) für die Verschiebung des Schienbeins, der gegen die obere vordere Seite des Schienbeins bzw. gegen die TTA (Tibial Tuberosity Advancement) zur Anlage kommt,
**dadurch gekennzeichnet,**
**dass** sie Mittel zum Festspannen des Glieds an der Stütze umfasst, wobei die Festspannmittel Mittel zur Messung und/oder zur Erkennung einer Spannkraft umfassen.

2. Vorrichtung nach Anspruch 1,
**dadurch gekennzeichnet,**
**dass** sie mindestens eine Oberflächenelektrode gegenüber den Oberschenkelmuskeln und eine Referenzelektrode am seitlichen oder mittigen Knierand aufweist, um jegliche Muskel-Störkontraktion zu erfassen, die die genaue Feststellung des Ausmaßes der vorderen Verschiebung des Schienbeins verhindert.

3. Vorrichtung nach einem der Ansprüche 1 und 2,
**dadurch gekennzeichnet,**
**dass** das Schiebemittel mindestens einen Zylinder umfasst, der durch einen Schubrechner gesteuert wird.

4. Vorrichtung nach Anspruch 3,
**dadurch gekennzeichnet,**
**dass** sie einen Drucksensor zur Messung des durch das Glied auf die Stütze ausgeübten Drucks umfasst, wobei der Rechner so mit dem Drucksensor verbunden ist, dass der Zylinder in Abhängigkeit von Daten gesteuert wird, die von dem Drucksensor bereitgestellt werden.

5. Vorrichtung nach einem der Ansprüche 3 und 4,
**dadurch gekennzeichnet,**
**dass** der Verschiebungssensor dem Rechner Daten in elektronischer Form bereitstellt.

6. Vorrichtung nach einem der vorhergehenden Ansprüche,
**dadurch gekennzeichnet,**
**dass** die Festspannmittel Mittel zum Festgurten des Oberschenkelknochens des Glieds an der Stütze aufweisen, wobei mindestens ein Drucksensor auf der Stütze in der Nähe des Gurts so angeordnet ist, dass ein Ausmaß der vom Glied auf die Stütze ausgeübten Stützkraft geliefert wird.

7. Vorrichtung nach Anspruch 6,
**dadurch gekennzeichnet,**
**dass** sie eine Quadrizeps-Halteschale (9) umfasst, die mittels mindestens zweier Gurte mit der Stütze verbunden ist.

8. Vorrichtung nach einem der vorhergehenden Ansprüche,
**dadurch gekennzeichnet,**
**dass** die Festspannmittel Mittel zum Festgurten des Fußes des Glieds umfassen.

9. Vorrichtung nach Anspruch 8,
**dadurch gekennzeichnet,**
**dass** die Mittel zum Festgurten des Fußes gleitend auf der Stütze angebracht sind, so dass sie von den Mitteln zum Festgurten des Oberschenkelknochens beabstandet/ daran angenähert werden können.

10. Vorrichtung nach Anspruch 9,
**dadurch gekennzeichnet,**
**dass** die Stütze Markierungen aufweist, entlang deren die Mittel zum Festgurten des Fußes gleitend angebracht sind.

11. Vorrichtung nach einem der Ansprüche 8 bis 10,
**dadurch gekennzeichnet,**
**dass** die Mittel zum Festgurten des Fußes Mittel zum Erkennen der Spannstärke aufweisen.

12. Vorrichtung nach einem der vorhergehenden Ansprüche,
**dadurch gekennzeichnet,**
**dass** sie Mittel zum Biegen (x) des Knies des Glieds umfasst, womit 15[deg.] < x < 25[deg.], wobei die Vorrichtung vorzugsweise einen Bereich zum Stützen des Oberschenkels und einen Bereich zum Stützen der Wade umfasst, welche miteinander verbunden sind und jeweils ein hinteres Ende und ein vorderes Ende aufweisen, wobei mindestens das vordere Ende Mittel für die Höheneinstellung aufweist.

13. Vorrichtung nach einem der vorhergehenden Ansprüche,
**dadurch gekennzeichnet,**
**dass** der Verschiebungssensor (13) an einem Galgen befestigt ist, der auf der Stütze gleitend und angelenkt montiert ist.

14. Vorrichtung nach einem der von Anspruch 2 abhängigen Ansprüche 3 bis 13,
**dadurch gekennzeichnet,**
**dass** die Elektroden mit Behandlungsmitteln verbunden sind, die an den Rechner gekoppelt sind, wobei die Behandlungsmittel dazu bestimmt sind, eine Neutralisierung des Schiebemittels zu bewirken, wenn die Elektrode(n) eine Muskelkontraktion erfassen.

15. Vorrichtung nach einem der von Anspruch 2 abhängigen Ansprüche 3 bis 14,
**dadurch gekennzeichnet,**
**dass** die Elektroden mit Mitteln zur Anzeige der Muskelkontraktion verbunden sind.

## Claims

1. Device for detecting and monitoring an injury (break or repair) of the ACL by anterior translation of the tibia relative to the femur comprising a support of the lower limb, a thrust means (2) for the posterosuperior face of the calf and a sensor (13) of displacement of the tibia coming to be positioned on the anterosuperior face of the tibia, on the ATT (Anterior Tibia Tuberosity), **characterized in that** it comprises means for clamping said limb on said support, said clamping means comprising means for measuring and/or identifying a clamping force.

2. Device according to Claim 1, **characterized in that** it comprises at least one surface electrode facing the hamstring muscles and a reference electrode level with the lateral or medial edge of the knee making it possible to detect any stray muscular contraction which prevents the measurement of the anterior displacement of the tibia from being determined accurately.

3. Device according to one of Claims 1 and 2, **characterized in that** said thrust means comprises at least one actuator driven by a thrust computer.

4. Device according to Claim 3, **characterized in that** it comprises a pressure sensor intended to measure the pressure exerted by said limb on said support, said computer being linked to the pressure sensor so as to drive said actuator according to data supplied by said pressure sensor.

5. Device according to one of Claims 3 and 4, **characterized in that** said displacement sensor supplies data in electronic form to said computer.

6. Device according to any one of the preceding claims, **characterized in that** said clamping means comprise means for strapping the femur of said limb onto said support, at least one pressure sensor being placed on said support in the vicinity of said strap so as to obtain a measurement of the bearing force of said limb on said support.

7. Device according to Claim 6, **characterized in that** it comprises a quadricipital securing shell (9) linked by at least two straps to said support.

8. Device according to any one of the preceding claims, **characterized in that** said clamping means comprise means for strapping the foot of said limb.

9. Device according to Claim 8, **characterized in that** said means for strapping the foot are mounted to slide on said support so as to be able to be moved away from/closer to said femur strapping means.

10. Device according to Claim 9, **characterized in that** said support has graduations along which said foot strapping means are mounted to slide.

11. Device according to one of Claims 8 to 10, **characterized in that** said foot strapping means bear means for identifying the clamping level.

12. Device according to any one of the preceding claims, **characterized in that** it comprises means for flexing (x) the knee of said limb making it possible to have: 15[deg.]< x < 25[deg.],said device preferentially comprising a thigh support part and calf support part linked together, and respectively having a rear end and a front end, at least said front end having height adjustment means.

13. Device according to any one of the preceding claims, **characterized in that** said displacement sensor (13) is fixed to a boom mounted on the support in a sliding and articulated manner.

14. Device according to one of Claims 3 to 13, dependent on Claim 2, **characterized in that** said electrodes are linked to processing means coupled to said computer, said processing means being intended to provoke a neutralization of said thrust means in case of detection of muscular contraction by said electrode or electrodes.

15. Device according to any one of Claims 3 to 14 dependent on Claim 2, **characterized in that** said electrodes are linked to means for displaying said muscular contraction.
